# EUROPEAN PATENT APPLICATION

(11) **EP 4 684 791 A2**
(43) Date of publication of application: **28.01.2026**
(21) Application number: 25191487.5
(22) Date of filing: 24.07.2025
(51) Int. Cl.: A61K 35/60, A61K 8/60, A61K 31/711, A61P 19/02, A61P 19/10

(54) **METHOD OF EXTRACTING DNA FROM FISH AND METHOD OF USING THE SAME IN OSTEOPOROSIS AND OSTEOARTHRITIS**

(30) Priority: 26.07.2024 US 202463675989 P
(71) Applicant: Lytone Enterprise, Inc., New Taipei City 221-80 (TW)
(72) Inventor: LU, MING JHEN, 221-80 NEW TAIPEI CITY (TW); HAN, YU-TSUNG, 221-80 NEW TAIPEI CITY (TW); CHANG, WEI-TING, 221-80 NEW TAIPEI CITY (TW); CHANG, WILLIAM T.H., 221-80 NEW TAIPEI CITY (TW)
(74) Representative: Straus, Alexander

(57) **Abstract**

The present disclosure provides a method of extracting DNA from fish and a method of using the DNA extract. The DNA extract provided herein produces the efficacies in ameliorating or treating osteoarthritis or osteoporosis. Mice experiments prove that clinical parameters associated with osteoarthritis or osteoporosis are improved by the DNA extract. In addition, the DNA extract exhibits free radical scavenging activities, anti-oxidative activities, anti-depressant activities, and black hair promoting activities.

## Description

### Field of the Invention

The invention relates to a method of extracting DNA from fish, particularly from fish testes, and a method of using the DNA extract, especially by oral administration in osteoporosis and osteoarthritis.

### Background of the Invention

DNA mixture purified from salmon sperm DNA is known to exhibit several pharmacological activities. Such DNA mixture is generally known as the polydeoxyribonucleotide (PDRN). PDRN contains a mixture of deoxyribonucleotides polymers ranging between 50 and 2000 bp (Pizzino et al., Adenosine Receptor Stimulation Improves Glucocorticoid-Induced Osteoporosis in a Rat Model, Frontiers in Pharmacology, 05 September 2017, Volume 8, Article 558). PDRN is a registered drug that exhibits tissue repairing, anti-ischemic, and anti-inflammatory activities. These activities suggest the therapeutic uses of PDRN in regenerative medicine and in diabetic foot ulcers (Squadrito et al., Pharmacological Activity and Clinical Use of PDRN, Frontiers in Pharmacology, 26 April 2017, Volume 8, Article 224). Another type of DNA mixture purified from salmon contains DNA of higher base pairs, for example, more than 20,000 bp. This type of DNA mixture is reported as being obtained from salmon testes and having activities for bone regeneration (Sato A, Kajiya H, Mori N, Sato H, Fukushima T, et al. (2017) Salmon DNA Accelerates Bone Regeneration by Inducing Osteoblast Migration. PLOS ONE 12(1): e0169522). The DNA mixture is prepared as a DNA disk and implanted into a critical-size calvarial bone defect (CSD). The disk is biodegraded within 3 days after implantation. However, the DNA disks induces osteogenic cells, including both MSC-like and osteoblast-like cells, migration to the defect site to replace the fibrous connective tissue in the bone defect. New bone formation is observed in the defect receiving the DNA disk under micro-CT scans. The results of the H&E-stain are in agreement with the new bone regeneration observed in samples from the DNA disk-implantation by micro-CT findings.

Also known is a commercially available PDRN product, the PRF001 (PRP salmon DNA; Pharma Research Product, Gangneung, Korea), which is a fragmented DNA polymer extracted from the testes of adult chum salmons and having molecular weights of 50 to 1,500 kDa. The PDRN product is reported as having anti-inflammatory effects *in vitro* and protective effect against osteoarthritis in rat model (Ra HJ, et al. Effects of salmon DNA fraction in vitro and in a monosodium iodoacetate-induced osteoarthritis rat model. Korean J Physiol Pharmacol. 2018 Mar;22(2):163-172). It is reported that *in vitro,* the levels of inflammatory markers, IL-1β, TNF-α, COX-2, iNOS, and PGE2, were significantly suppressed by PRF001 treatment. *In vivo,* the inflammatory mediators and cytokines, IL-1β, p-Erk1/2, NF-kB, TNF-α, COX-2, and PGE2, as well as MMP3 and MMP7, were decreased in the monosodium iodoacetate (MIA)-induced osteoarthritic rats following intake of PRF001. The anti-inflammatory property of PRF001 is considered to contributes to its protective effects in osteoarthritis through regulating the cytokines/signal transducers.

PDRN is also reported to yield similar function as hyaluronic acid (HA) injection in relieving knee osteoarthritis pain when administered intra-articularly while avoiding common HA side effects (Kim MS, Cho RK, In Y. The efficacy and safety of polydeoxyribonucleotide for the treatment of knee osteoarthritis: Systematic review and meta-analysis of randomized controlled trials. Medicine (Baltimore). 2019 Sep;98(39):e17386). The pain-relief benefits of PDRN in clinical practice could offer a complementary role for other injection treatments.

Food grade PDRN products are known in the art. For example, a commercially available food-PDRN product, the PRF002 (PRP salmon DNA; Pharma Research Product, Gangneung, Korea), is reported as being capable of ameliorating alcohol-induced gastric mucosa damage by oral administration (Kim J, et al., Amelioration of alcohol-induced gastric mucosa damage by oral administration of food-polydeoxyribonucleotides. Mol Med Rep. 2021 Nov;24(5):790). The food-PDRN product is recommended to be taken regularly as a food supplement for beneficial effects. In addition, oral administration of PDRN may inhibit the expression of IL-1β, matrix metalloproteinase (MMP)-3, and MMP-7, as well as production of inflammatory mediators including COX-2, prostaglandin E2 (PGE2), and TNF-α. Oral administration of PDRN also exerts a mucoprotective effect on IND-induced gastropathy (Tae-Hee Kim et al., Applications of Marine Organism-Derived Polydeoxyribonucleotide: Its Potential in Biomedical Engineering Mar. Drugs 2021, 19(6), 296).

However, there remains a need for novel DNA extracts from fish and development of medical applications of such DNA extracts.

### Summary of the Invention

The present disclosure provides a novel process for extracting DNA from fish testes, DNA extract obtained from the process, and methods of treating or ameliorating osteoarthritis or osteoporosis, removing free radicals, reducing oxidative stress, treating or ameliorating depression, and promoting black hair formation, using such DNA extract.

In an embodiment, the fish used in the extraction process is a deep sea fish, for example, mackerel or salmon. In a preferred embodiment, the fish is mackerel.

In an embodiment, the fish testes are homogenized to obtain a homogenate. In an embodiment, the fish testes are frozen and thawed before the homogenization step. In the embodiment where the fish testes are frozen, the frozen temperature is -10 to -80 °C. In a preferred embodiment, the frozen temperature is -10 to -40°C. In a preferred embodiment, the frozen temperature is -10 to -20°C.

In an embodiment, the fish testes are washed by a weak acid before the homogenization step. In a preferred embodiment, the weak acid is citric acid.

In an embodiment, the homogenate is incubated with an acid. In a preferred embodiment, the acid is HCl. In a more preferred embodiment, the HCl is 0.01-1 M HCL, preferably 0.05-0.5 M HCl. In a preferred embodiment, the incubation is 30-240 min, preferably 30-180 min.

In an embodiment, the homogenate is subjected to centrifugation to collect a pellet. In a preferred embodiment, the centrifugation is at 20 g-15000 g for 30 sec-90 min, preferably 30 g-12000 g for 15-60 min.

In an embodiment, the pellet is re-suspended with water and the pH of the suspension is adjusted to pH 6.0 to 9.0, preferably pH 7.0 to 8.0. In a preferred embodiment, the ratio of pellet and water is 1:1 to 1:15, preferably 1:5 to 1:12, based on weight.

In an embodiment, the suspension is heated. In a preferred embodiment, the heating is 60-150°C, preferably 70-130°C for 5-150 min, preferably 15-120 min.

In an embodiment, the suspension is cooled after heating and added with a proteinase and/or a nuclease to form a mixture. In a preferred embodiment, the proteinase and/or nuclease is one or more of alcalase, flavourzyme, protinase, nuclease, pepsin and trypsin. In a more preferred embodiment, the proteinase and/or nuclease is trypsin. In a preferred embodiment, the proteinase and/or nuclease is incubated with the suspension for 0.5-48 hr, preferably 0.5-24 hr.

In an embodiment, the proteinase and/or nuclease is inactivated by heating. In a preferred embodiment, the proteinase and/or nuclease is inactivated by heating under 50-120°C, preferably 70-100°C for 10-70 min, preferably15-60 min.

In an embodiment, the DNA extract is obtained by separating the mixture to obtain a supernatant containing the DNA. In a preferred embodiment, the separation is filtration or centrifugation.

In an embodiment, the DNA extract is subjected to a step of removing fishy smell. In a preferred embodiment, the fishy smell is removed by diatomaceous earth. In a preferred embodiment, the fishy smell is removed by cyclodextrin.

The present disclosure relates to DNA extract produced by the process described above. In an embodiment, the DNA extract comprises DNA molecules having DNA base pairs between 10 and 2000 bp, preferably between 10 and 500 bp, more preferably between 50 and 250 bp.

The present disclosure further relates to methods of using the DNA extract. In an embodiment, the disclosure provides a method of treating or ameliorating osteoarthritis or osteoporosis in a subject in need thereof, comprising administering to the subject a therapeutically effective amount of the DNA extract. In an embodiment, administration of the DNA extract reduces osteoarthritis pain. In a preferred embodiment, the DNA extract reduces one or more of inflammation, pannus formation, cartilage damage, and bone resorption. In an embodiment, administration of the DNA extract increases limb supporting force. In an embodiment, administration of the DNA extract increases trabecular bone formation, surface area, and/or thickness.

In an embodiment, the disclosure provides a method of removing free radicals in a subject in need thereof, comprising administering to the subject a therapeutically effective amount of the DNA extract. In a preferred embodiment, the free radical is NO free radical.

In an embodiment, the disclosure provides a method of reducing oxidative stress in a subject in need thereof, comprising administering to the subject a therapeutically effective amount of the DNA extract.

In an embodiment, the disclosure provides a method of treating or ameliorating depression in a subject in need thereof, comprising administering to the subject a therapeutically effective amount of the DNA extract. In a preferred embodiment, administration of the DNA extract inhibits monoamine oxidase (MAO).

In an embodiment, the disclosure provides a method of promoting black hair formation in a subject in need thereof, comprising administering to the subject a therapeutically effective amount of the DNA extract. In a preferred embodiment, administration of the DNA extract increases tyrosinase activities.

In an embodiment, the DNA extract in the methods described above is administered to a subject orally. In a preferred embodiment, the oral administration is in the solid form, such as a tablet, a capsule, a powder, or a granule, or in the form of a solution, such as a suspension, a solution, or an emulsion. In an embodiment, the DNA extract is used as food supplement.

The present invention is described in detail in the following sections. Other characterizations, purposes and advantages of the present invention can be easily found in the detailed descriptions and claims of the invention.

### Brief Description of the Drawing

Fig. 1 shows the results of 1.5% agarose gel electrophoresis of DNA extracts from mackerel hydrolyzed by trypsin for 1 to 6 hours and salmon DNA extract.
Fig. 2 shows the animal experiment procedure.
Fig. 3 shows body weight changes of animals during the experiment.
Fig. 4 shows the scores of erythma or edema in animals after MIA treatment. The data of each group are shown as Mean ± SEM. The scores of erythma or edema of all animals in the same group are summed and divided by animal number.
Fig. 5 shows the images of knee joints after topical stimulation. Scale bar = 1.0 cm. The numbers in parenthesis below each image represent animal numbering.
Fig. 6 shows the threshold of pain tolerance in the joint. The maximal stress value that can be tolerated in the right hind limb (the diseased limb) after administration at two and four weeks are demonstrated. The data are shown as Mean ± SEM, *: p<0.05, compared to the G1 (OVX+MIA+water) group (statistical analysis by Student t-test).
Fig. 7 shows the changes of hind limb supporting forces. The supporting forces of the left and right hind limbs are deducted by each other to obtain the variation value. The data are shown as Mean ± SEM, *: p<0.05, compared to the G1 (OVX+MIA+water) group (statistical analysis by Student t-test).
Fig. 8 shows the images of micro-tomography of the thigh bone. The degree of osteoporosis can be determined by the degree of bone mass density. Scale bar = 2.0 mm. The numbers in parenthesis below each image represent animal numbering.
Fig. 9 shows results of analysis of the images of micro-tomography of the thigh bone for bone mass density (A), bone surface area ratio (B), bone volume ratio (C), bone surface area density (D), trabecular thickness (E), and trabecular number (F). The data are shown as Mean ± SEM, *: p<0.05, significant change compared to the G1 (OVX+MIA+water) group (statistical analysis by Student t-test).
Fig. 10 shows the images of micro-tomography of tissue in the knee joints. The images are 50X and show the results of H&E stain. The numbers in parenthesis below each image represent animal numbering.
Fig. 11 shows the results of analysis of tissue in the knee joints. The evaluated items include total scores of osteoarthritis (A) and inflammation, pannus formation, cartilage damage, and bone resorption for osteoarthritis histopathology analysis (B). The data are shown as Mean ± SEM, *: significant change compared to the G1 (OVX+MIA+water) group (p<0.05, Student t-test). L: Composition L, M: Composition M, H: Composition H, and X: Composition X.
Fig. 12 shows the results of analysis of tissue of the femurs. The evaluated items include: trabecular thickness, and trabecular area. The data are shown as Mean ± SEM, *: significant change compared to the G1 (OVX+MIA+water) group (p<0.05, Student t-test). L: Composition L, M: Composition M, H: Composition H, and X: Composition X.
Fig. 13 shows the images of micro-tomography of tissue in the femurs. The images are 50X and show the results of H&E stain. The numbers in parenthesis below each image represent animal numbering.
Fig. 14 shows the efficacies of the DNA extracts to clear NO free radicals.
Fig. 15 shows the efficacies of the DNA extracts to chelate ferrous ions.
Fig. 16 shows the efficacies of the DNA extracts to inhibit monoamine oxidase (MAO).
Fig. 17 shows the efficacies of the DNA extracts to increase tyrosinase activities.

### Detailed Description of the Invention

For the purpose of a more clear understanding of the technical features, goals and advantages of the present invention, provided below are detailed descriptions of the technical schemes of the present invention. The example is offered for illustrative purposes only, and is not intended to limit the scope of the present invention in any way.

### Examples

### Example 1: Extraction of DNA From Mackerels

Freshly collected testes from mackerels were frozen in -10 to -80°C and thawed under room temperature before use. The thawed testes were washed with citric acid and subjected to homogenization to obtain a chylous or pulpy homogenate. The homogenate was added with 0.05M-0.5M HCl and incubated for 30-180 min. After then, the mixture was subjected to centrifugation under 30-12000 g for 15-60 min. The supernatant was removed by filtration. The pellet was collected and added with distilled water (solid : water ratio = 1:5-1:12, based on weight) to re-suspend the pellet. The pH of the suspension was adjusted to pH~7.0-8.0. After then, the suspension was heated under 70-130°C for 30-120 min for sterilization. After cooling, the suspension was added with trypsin, and then incubated under 10-40°C for 0.5-24 hr. The enzyme was inactivated by heating under 70-100°C for 15-60 min. After enzyme inactivation, the mixture was subjected to filtration or centrifugation under 30-12000 g for 15-60 min. The supernatant containing the DNA extract was collected. To remove the fishy smell, each unit (100-1000 mL) of the supernatant was treated with 0.02-60% diatomaceous earth and/ or cyclodextrin. The results of removing the fishy smell was evaluated by olfactory analysis. Supernatants passing the analysis were freeze-dried and stored under room temperature.

### Example 2: Characterization of the DNA Extracts

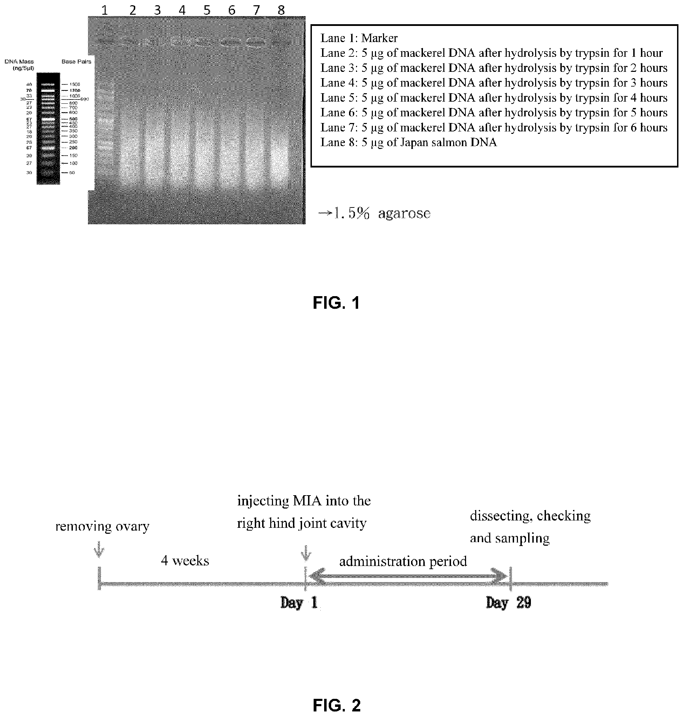

Analysis of the DNA obtained from Example 1 for DNA size was conducted. The DNA extracts were treated with trypsin for 1-6 hr. After trypsin treatment, the extracts were subjected to 1.5% agarose gel electrophoresis and absorbance at OD₂₆₀ was detected to evaluate DNA concentrations. DNA obtained from salmon according to the extraction procedure of Example 1 was used for comparison. The results are shown in Figure 1 which demonstrates that the distribution of DNA base pairs is between 10 and 500 bp. The range of DNA base pairs overlaps with the range between 50 and 2,000 bp known for DNA.

The DNA extracts were further evaluated for DNA purity. The supernatants containing mackerel DNA extracts that were subjected to different time periods of trypsin digestions as described above were detected for OD₂₆₀ and OD₂₈₀ values. The results are shown in Table 1 below.

| Table 1. Effect of Trypsin Digestion Time on DNA Purity of Mackerel DNA Extracts | | |
|---|---|---|
| Digestion Time | 1-3 hr | 4-6 hr |
| OD₂₆₀ | 0.096-0.112 | 0.111-0.223 |
| OD₂₈₀ | 0.059-0.062 | 0.069-0.079 |
| OD₂₆₀/OD₂₈₀ | 1.65-1.80 | 1.62-1.70 |

From the results of OD₂₆₀/OD₂₈₀ shown in Table 1, it is found that the values of OD₂₆₀/OD₂₈₀ are between 1.60-1.70.

### Example 3: Removal of Fishy Smell From the DNA Extracts

### Example 3.1: Removal of Fishy Smell By Using Diatomaceous Earth (DE) and/ or Cyclodextrin (CD)

DNA extracts after trypsin digestion were added with different amounts of DE and/ or CD, and incubated under room temperature or 30-60°C for 18-36 hr followed by centrifugation. The supernatants were collected and evaluated for the efficacies to removed fishy smell by olfactory analysis. The test design is shown in Table 2 below.

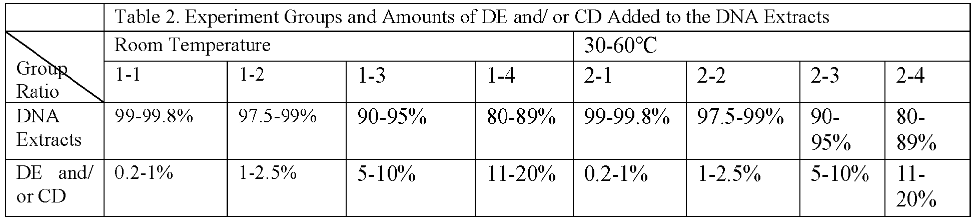

In the olfactory analysis, at least two test persons evaluate the fishy smell. The results are shown in Table 3 below which shows that addition of 5-20% DE and/ or CD can remove the fishy smell.

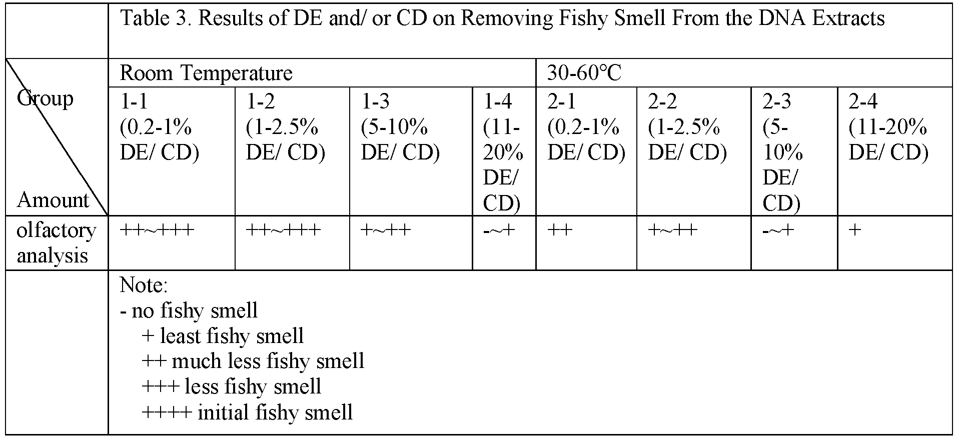

### Example 4: Evaluations of the DNA Extracts for Efficacies in Osteoporosis and Osteoarthritis in Animal Model

### 4.1 Experimental animals grouping and treatments

Test SD female rats were subjected to removal of ovaries (performed by BioLASCO, Taiwan) and transferred to animal rooms for recovery and adaptation for 4 weeks. The rats were weighed and grouped into 5 groups (Groups 1 to 5) based on body weights. Each groups has 5 female rats. After grouping, the body weights of the animals do not have significant difference among groups (P < 0.05). Five additional female rats having the same age as the grouped animals and subjected to the same surgical operation without removing the ovaries were grouped as the Sham control group (Group 0).

**Table 4: Experimental groups**

| **Animal Grouping** | **Treatment** | **Dose (mg/kg)** | **Concentration (mg/mL)** | **Volume (mL/kg)** | **Number of Female Rats** |
|---|---|---|---|---|---|
| 0 | Sham+water | 0 | 0 | 10 | 5 |
| 1 | OVX+MIA+water | 0 | 0 | 10 | 5 |
| 2 | OVX+MIA+L | 281.25 | 28.125 | 10 | 5 |
| 3 | OVX+MIA+M | 312.5 | 31.25 | 10 | 5 |
| 4 | OVX+MIA+H | 343.75 | 34.375 | 10 | 5 |
| 5 | OVX+MIA+X | 62.5 | 6.25 | 10 | 5 |

**Table 5: Contents of treatment**

| Animal Grouping | Test Compositions | Contents of Test Compositions (human dose) (%)* |
|---|---|---|
| 0 | water | water |
| 1 | water | water |
| 2 | L | 43.52% fish collagen + 43.52% N-acetylglucosamine + 12.96% fish scale extract (75% Hydroxyapatite, HAP) |
| 3 | M | 39.17% fish collagen + 39.17% N-acetylglucosamine + 11.67% fish scale extract (75% Hydroxyapatite, HAP) + low dose (10%) salmon DNA extracts |
| 4 | H | 35.61% fish collagen + 35.61% N-acetylglucosamine + 10.61% fish scale extract (75% Hydroxyapatite, HAP) + high dose (18.18%) salmon DNA extracts |
| 5 | X | high dose (100%) salmon DNA extracts |

| | | |
|---|---|---|
| *The human dose of each test composition is converted into rat dose according to the equation: rat daily dose = (human daily dose/60 kg)*6.25 | | |

### 4.2 Experiment procedure

After removal of ovaries and adaptation for 4 weeks, rats were injected with MIA (monosodium iodoacetate) into the knee joint of the right hind limb on Day 1. The treatments were performed from Day 1 to Day 29. After then, the rats were sacrificed and sampled. A flow chart of the experiment procedure is shown in Figure 2.

### 4.2.1 Induction of osteoarthritis (OA)

On the first day of the experiment, female rats of Groups 1 to 5 were subjected to removal of ovaries. Each animal was anaesthetized using isoflurane followed by injection of 50 µL MIA saline (4 mg/50 µL) into the right knee joint by using 26G needle. After completion, the rats were returned to the cages for recovery. The animals of Group 0 were subjected to the same operation but injected with normal saline for use as normal control.

### 4.2.2 Administration of test compositions

Starting from the day of MIA induction (Day 1), the rats were orally fed (gavage) with water or test compositions once a day for 28 days. The first day of administration is defined as "the first day of experiment" or D1 (Day 1).

### 4.3 Experimental results

### 4.3.1 Observation on animals (SOP-TM201)

During experiment, animals were observed once a day. Only swelling of the knee joints was observed three days (~Day 3) after injection of MIA for inducing OA during the experiment. However, it is the toxic response known to be induced by MIA. Besides, no other abnormality was found.

### 4.3.2 Animal body weights (Precisa, XB6200D)

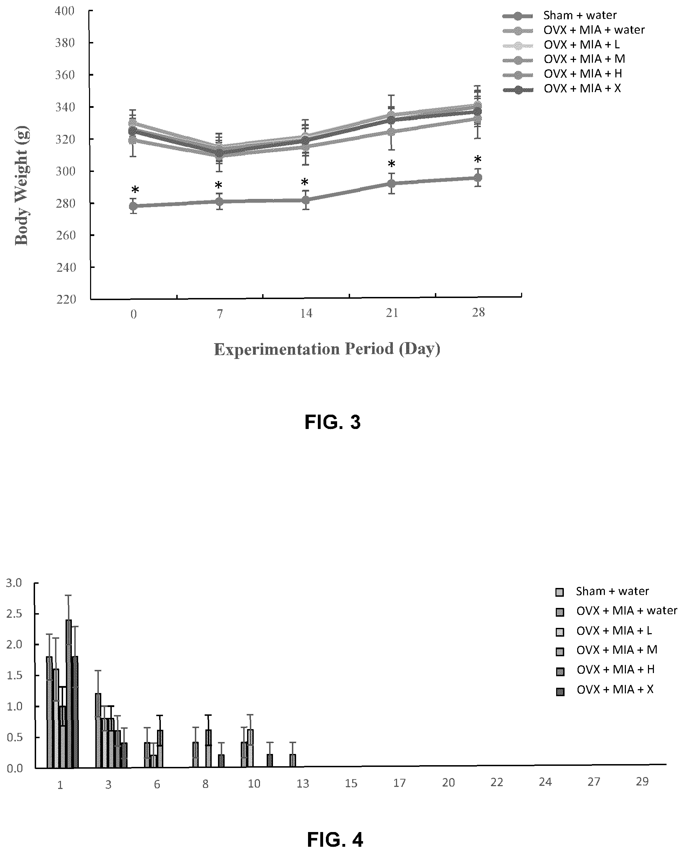

During experiment, the animals were weighed once weekly. After removal of ovaries from the female rats, due to changes in hormone, the body weights of animals of Group 1 to Group 5 are significantly higher than those of the animals of Group 0. During the experiment, the body weights of Group 1 have no difference compared to Group 2 to Group 5. The growth curves of each group are similar (Figure 3).

### 4.3.3 Evaluation of OA topical responses

During experiment (after MIA induction), evaluation of erythma or edema at the right knee was conducted through observation three times a week.

| **Table 6. Symptom Severity Score** | | |
|---|---|---|
| **Erythma and Eschar Formation** | **Numerical grading** | **Edema formation** |
| No erythema | 0 | No edema |
| Very slight erythema (barely perceptible) | 1 | Very slight edema (barely perceptible) |
| Well-defined erythema | 2 | Well-defined edema (edges of area well-defined by finite raising) |
| Moderate erythema | 3 | Moderate edema (raised approximately 1mm) |
| Severe erythema (beet-redness) to eschar formation | 4 | Severe edema (raised more than 1mm and extending beyond exposure area) |

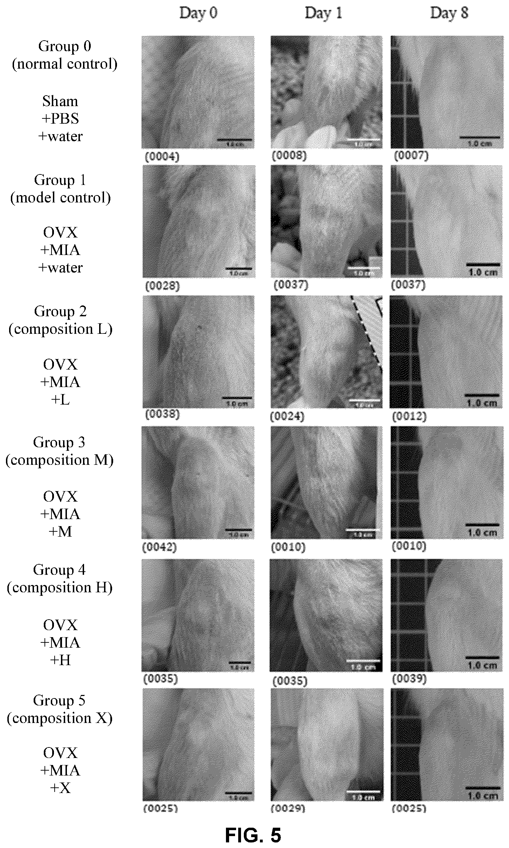

The topical stimulus response at the injection site of MIA can be seen in Figure 4. Photos of the topical site are shown in Figure 5.

From observation through naked eyes, it can be found that injection sites at the joint showed certain degree of topical stimulus response: in Day 1, each group treated with MIA showed a score of 1.0~2.4, indicating a medium to low degree of stimulus response of MIA at the joint cavity; in Day 3, stimulus responses of all groups decreased compared to Day 1. The average score of stimulus response of G1 (OVX+MIA+water), G2 (OVX+MIA+L), G3(OVX+MIA+M), G4 (OVX+MIA+H) and G5 (OVX+MIA+X) are 1.0, 0.8, 0.8, 0.6 and 0.4, respectively, which are without statistical significance. For observation in later period of the experiment, the average scores of stimulus response are lower than 0.6 which is an negligible stimulus response in the evaluation system having a total score of 8. Thus, the test compositions can ameliorate the stimulus responses caused by MIA in a dose-dependent manner. The Sham group (G0: water) showed no topical stimulus response throughout the experiment period.

### 4.3.4 Evaluation on osteoarthritis (OA) pain

Before administration of the test compositions (Week -1) and after administration for two and four weeks, measurements for the pain index, i.e., the gf (gram-force) value, of the right hind limb were performed by using a pressure application measurement (PAM) device (Ugo Basile, Italy, Cat. No.: 38500).

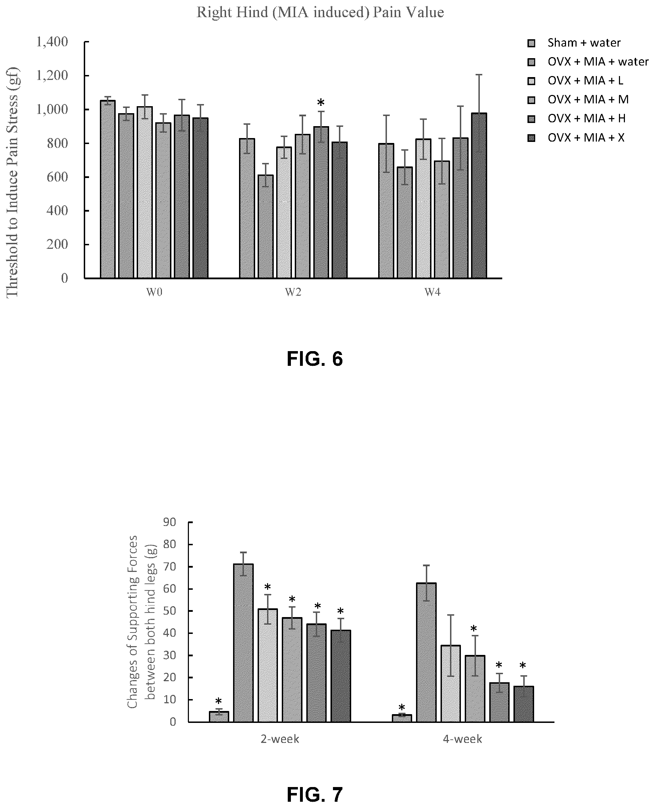

The evaluation results of the test compositions on osteoarthritis pain induced by MIA are shown in Figure 6.

Press the muscle around the knee joint by the PAM device to measure the threshold that induces arthritis pain in the rats. Lower threshold represents higher pain index value (more pain) and higher threshold represents lower pain index value (less pain). Before the experiment, the threshold to induce arthritis pain for the animals of each group falls between 920 and 1152 gf. After injecting water or MIA solution into the knee joints, the threshold decreased which indicates that injections into the joint cavity induce pain. After MIA injection into the joint cavity and two weeks after treatment with control composition or test compositions, the average threshold of pain for each group [G2 (OVX+MIA+L): 776.40±65.79, G3(OVX+MIA+M): 851.661112.86, G4 (OVX+MIA+H): 879.28±90.33 and G5 (OVX+MIA+X): 806.16±94.63] is higher than that of the disease control group [G1 (OVX+MIA+water): 612.12±68.40], wherein the difference between G4 and G1 reaches statistical significance. After MIA injection into the joint cavity and four weeks after treatment with control composition or test compositions, the average threshold of pain for each group (G2: 824.20±18.57, G3: 695.00±134.54, G4: 831.44±188.05 and G5: 806.16±94.63) is higher than that of the disease control group (G1: 976.80±228.94), however, without statistical significance. Based on the above results, it is found that the test composition (OVX+MIA+H) can ameliorate arthritis pain induced by MIA.

### 4.3.5 Evaluation on hind limb supporting force

At two and four weeks after administration of the test compositions, the supporting force (unit: g) of hind limb was measured using Librae Incapacitance Tester for rats (WP, Taiwan) as an indicator of arthritis pain.

The results of the evaluation of hind limb supporting force are shown in Figure 7.

By measuring the differences between the weights that both hind limbs can support, the variation of the supporting forces of both hind limbs caused by reducing the load of the right hind limb for relieving the pain after the right hind limb is induced with arthritis is evaluated. If the limb with arthritis can support a weight similar to that supported by a normal limb, the test composition is considered having therapeutic efficacy on the arthritis limb. The disease control group (G1 : OVX+MIA+water) showed obviously increased differences in the supporting forces of both limbs compared to the normal control group (G0 Sham+PBS+water), and such differences reach statistical significance. Two and four weeks after induction of arthritis, the variations of the supporting forces of both hind limbs increased 15- and 20-folds, respectively, indicating successful induction of the arthritis model.

Two weeks after induction of arthritis and administration of test compositions, each test group showed obviously decreased variations of supporting forces of both limbs compared to the disease control group (G1) and the decreases reached statistical significance. The test groups G2 (OVX+MIA+L), G3(OVX+MIA+M), G4 (OVX+MIA+H) and G5 (OVX+MIA+X) relieved pain by 28%, 34%, 38% and 42%, respectively, compared to G1.

Four weeks after induction of arthritis and administration of test compositions, each test group showed obviously decreased variations of supporting forces of both limbs compared to the disease control group (G1:OVX+MIA+water). Except for G2, G3 to G5 reached statistical significance. The test groups G2, G3, G4 and G5 relieved pain by 45%, 52%, 72% and 74%, respectively, compared to G1.

### 4.3.6 Evaluation of thigh bone micro-computed tomography

The thigh bone of the left hind limb was fixed in fix solution (10% Formalin) and subjected to micro-tomography to evaluate bone parameters such as bone mass density and trabecular bone.

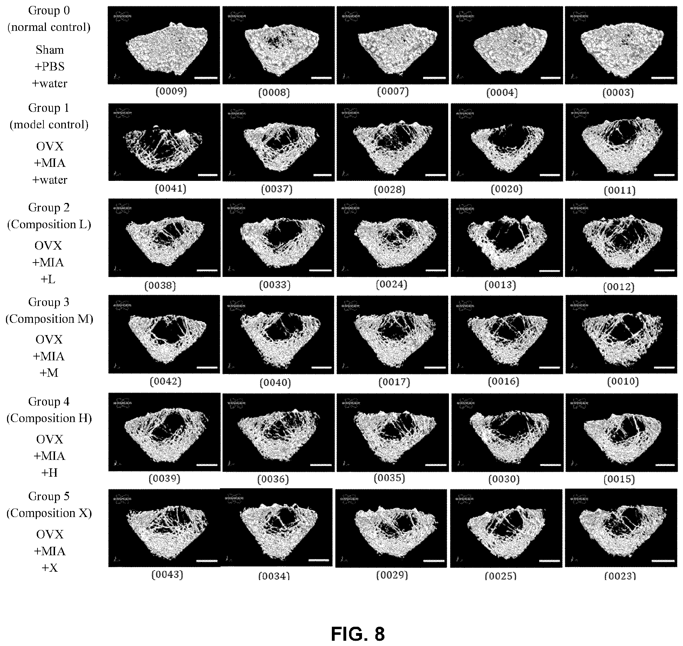

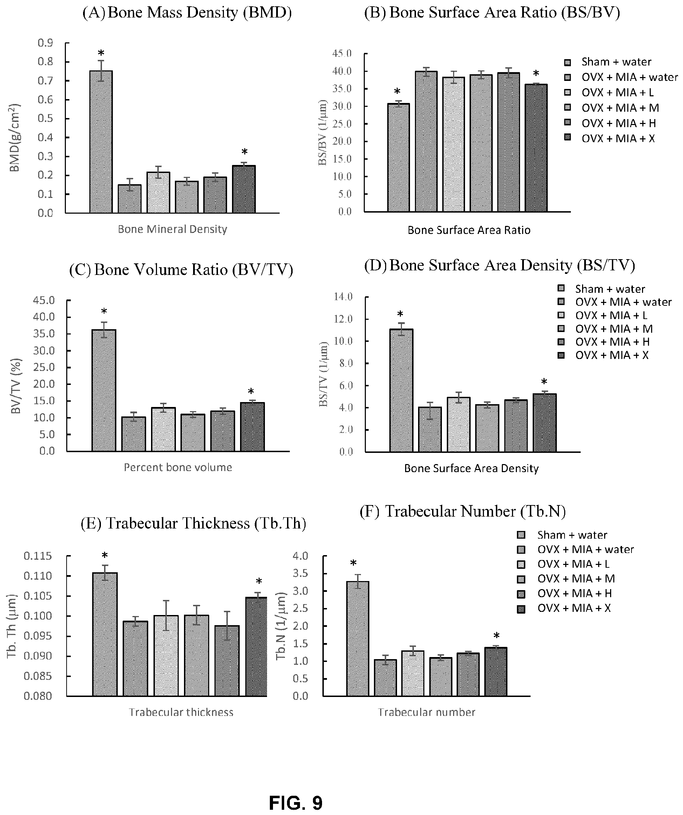

The images of thigh bone micro-tomography are shown in Figure 8. The results of the analysis of the images of the thigh bone micro-tomography are shown in Figure 9.

From the images of thigh bone micro-tomography in Figure 8, it can be directly found that the bone mass of thigh of the normal control group (G0) was dense with only tiny holes. In the osteoporosis control group (G1: OVX+MIA+water), the bone mass is loose with large holes and several dispersing trabecular bones. For test groups G2 to G5, the overall bone mass is more dense than G1, the holes are smaller, and the trabecular bones dispersed within the holes are more and apparent.

The images were further analyzed by micro-tomography. The results show that compared to the G0 normal control group, the bone mass density (BMD), bone volume percentage (BV/TV), bone surface area density (BS/TV), trabecular bone thickness (TB.TH), and number of trabecular bone (TB.N) of the osteoporosis control group G1 are 19.9%, 28.3%, 36.5%, 89.0% and 31.7%, respectively, of those of the GO group. In addition, the bone surface area ratio (BS/BV) of G1 is 130% of that of G0. All of the above results reach statistically significant difference. The results of the analysis are consistent with the images, indicating that the establishment of the osteoporosis disease model is successful.

Compared to the G1 disease model group, the average values of BMD, BV/TV, BS/TV, TB.TH and TB.N of G2, G3 and G4 are higher than those of G1 while the values of BS/BV are lower than that of G1. However, the above differences do not reach statistical significance. The above results indicate that the test compositions for G2 (OVX+MIA+L), G3(OVX+MIA+M) and G4 (OVX+MIA+H) may have potential in ameliorating osteoporosis.

Compared to the G0 normal control group, the values of BMD, BV/TV, BS/TV, TB.TH and TB.N of G5 are 167%, 141%, 130%, 106% and 133%, respectively, of those of G1 while the value of bone surface area ratio (BS/BV) is 90.7% of that of G1. All of the above differences reach statistical significance. The results prove that the test composition for G5 (OVX+MIA+X) has efficacy in treating osteoporosis.

### 4.3.7 Analysis of the histopathology of knee joint cavity

After completion of the experiment, live test animals were randomly subjected to euthanasia with carbon dioxide (CO₂) (SOP-TM210). Blood were collected, the corpses were dissected, and changes of the appearances and all the tissues and organs in the thoracic and abdominal cavities as well as the injection sites were checked with naked eyes and recorded. In addition, the left and right hind limbs were collected and fixed for preservation for subsequent treatments and evaluations.

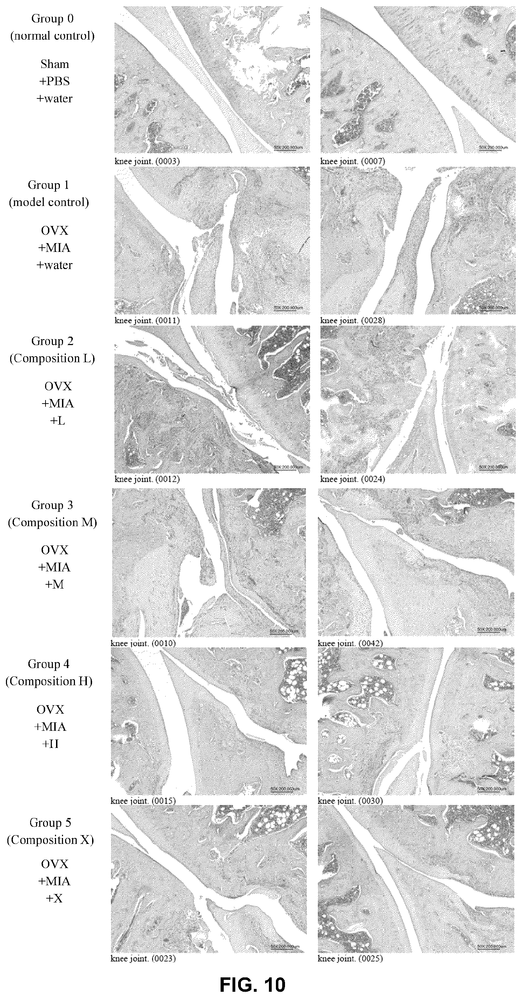

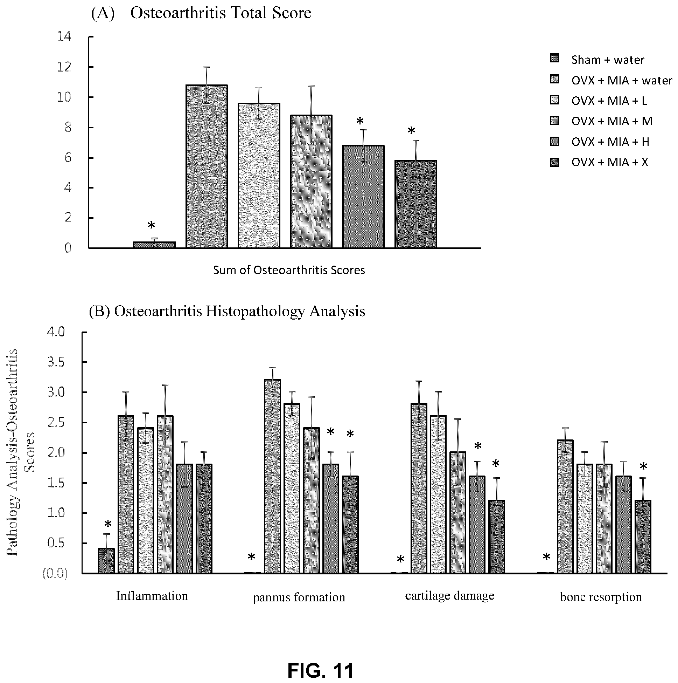

The tissue images of the knee joint cavity are shown in Figure 10. The results of the analysis of the histopathology of the knee joint cavity are shown in Figure 11.

The knees (including the flanking bones) of all the animals of the control and test groups were fixed, decalcified, trimmed, dehydrated, wax dipped, embedded, and sliced (4-6 µm) followed by H&E staining to prepare tissue slice biopsy. Analysis of tissue histopathology was conducted by professional veterinarian using microscope (Leica DM2700M, USA). The analysis of histopathology evaluated four arthritis indicators: inflammation, pannus formation, cartilage damage, and bone resorption. Each indicator was categorized into five grades according to severity : 0 - no response; 1- slight response; 2- low response; 3- medium response; 4- high and severe response.

The results of the analysis of the histopathology of knee joint cavity show that for the GO normal control group (Sham+PBS+water), only a negligible inflammatory response was found (average score 0.4). The other three indicators were scored 0. The total score of overall arthritis is also 0.4 which is determined negligible. Thus, the animals did not have arthritis and were healthy.

The scores of inflammation, pannus formation, cartilage damage, and bone resorption of the G1 disease model control group (OVX+MIA+water) are 2.6 (individual scores fall between slight response and severe response), 3.2 (individual scores fall between medium response and severe response), 2.8 (individual scores fall between slight response and severe response) and 2.2 (individual scores fall between slight response and medium response), respectively. The total score of arthritis is 10.8 which falls between slight and medium arthritis. Such score is about 27 times more severe than the normal control group, indicating that the arthritis induction model is successful.

The scores of inflammation, pannus formation, cartilage damage, and bone resorption as well as the total scores of arthritis of G2 (OVX+MIA+L) and G3 (OVX+MIA+M) are lower than those of G1. However, the differences do not reach statistical significance. The test compositions for G2 (OVX+MIA+L) and G3 (OVX+MIA+M) reduce arthritis responses by 11% and 19%, respectively.

The scores of inflammation, pannus formation, cartilage damage, and bone resorption of G4 (OVX+MIA+H) are 1.8, 1.8, 1.6 and 1.6, respectively. The total score of arthritis is 6.8. These scores are lower than those of G1. In addition, the scores of pannus formation and cartilage damage as well as the total score of arthritis are statistically significantly different from those of G1. The test composition for G4 (OVX+MIA+H) can significantly reduce arthritis responses by 37%.

The scores of inflammation, pannus formation, cartilage damage, and bone resorption of G5 (OVX+MIA+X) are 1.8, 1.6, 1.2 and 1.2, respectively. The total score of arthritis is 5.8. These scores are lower than those of G1. In addition, the scores of pannus formation, cartilage damage, and bone resorption as well as the total score of arthritis are statistically significantly different from those of G1. The test composition for G5 (OVX+MIA+X) can significantly reduce arthritis responses by 46%.

### 4.3.8 Analysis of the histopathology of femur

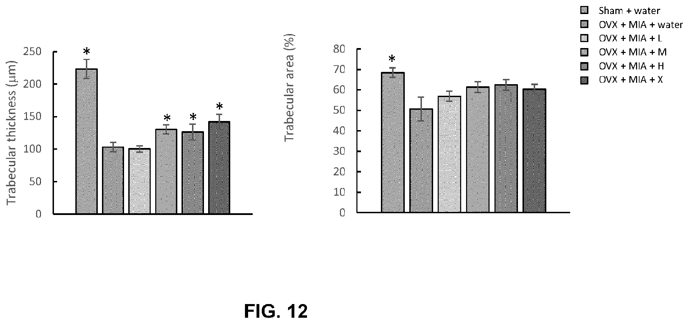

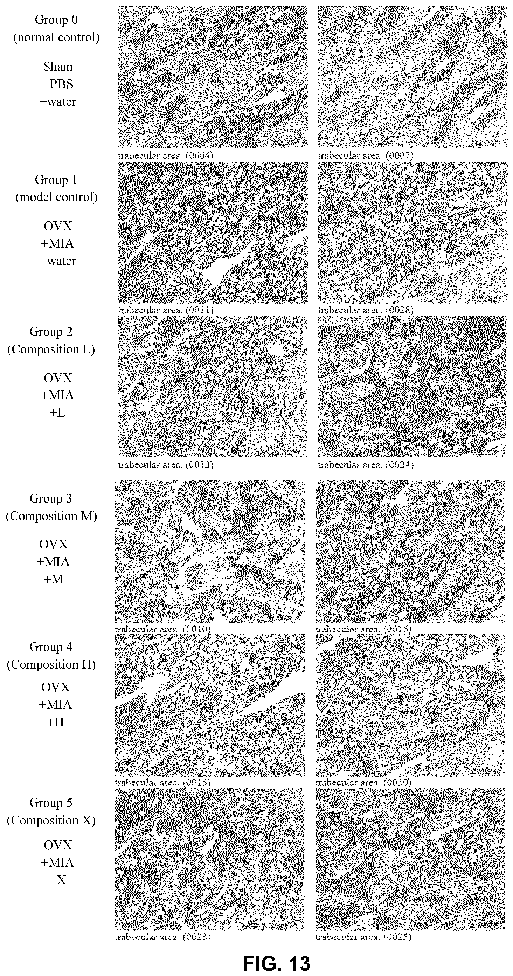

The results of the analysis of the histopathology of the femur are shown in Figure 12. The tissue images of the femur are shown in Figure 13.

The femur of the right hind limb of all the animals of the control and test groups were fixed, decalcified, trimmed, dehydrated, wax dipped, embedded, and sliced (4-6 µm) followed by H&E staining to prepare tissue slice biopsy. Analysis of tissue histopathology was conducted by professional veterinarian using microscope (Leica DM2700M, USA). The analysis of histopathology evaluated the morphology and microscopic structures of femur. The software ImageJ was used to analyze the thickness (µm) and surface (%) of the trabecular bones.

The results of the analysis of the histopathology of femur show that for the GO normal control group (Sham+PBS+water), a dense bone structure can be observed from the microscopic images of the femurs. The trabecular bones occupied 68.4% of surface area. The average thickness of the trabecular bones were 223 µm which is about 2.2 times of that of the G1 disease model control group (OVX+MIA+water). The results indicate that the femur is dense and pertains to a femur structure that an healthy animal should have.

For the G1 disease model group (OVX+MIA+water), from the microscopic images of femurs, it can be seen that the structure of the bone is loose. Numerous bubble-like structures are present between the trabecular bones. The trabecular bones occupied about 50.6% of surface area. This surface area is reduced by about 18%. The average thickness of the trabecular bones were 103 µm which is only about 0.5 times of that of the G0 normal control group. The results indicate osteoporosis of the femur. The osteoporosis induction model was successful. In addition, such degree of osteoporosis is suitable for use in evaluating the efficacies of healthy food.

For G2 (OVX+MIA+L), from the microscopic images of femurs, it can be seen that the structure of the bone is loose. Numerous bubble-like structures are present between the trabecular bones. The trabecular bones occupied about 56.8% of surface area. This surface area is increased by about 9.6% compared to G1. However, the average thickness of the trabecular bones were only 100 µm which is similar to that of G1. Thus, the test composition for G2 (OVX+MIA+L) does not exhibit effective ameliorative or therapeutic efficacy on osteoporosis.

For G3 (OVX+MIA+M), from the microscopic images of femurs, a bone structure better than those of G1 and G2 can be seen, though there were still bubble-like structures present between the trabecular bones. The trabecular bones occupied about 61.4% of surface area. This surface area is increased by about 10.8% compared to G1; however, without statistically significant difference. The average thickness of the trabecular bones were 130 µm which is about 1.3 times of that of G1 with statistical significance. Thus, the test composition for G3 (OVX+MIA+M) exhibits ameliorative or therapeutic efficacy on osteoporosis.

For G4 (OVX+MIA+H), from the microscopic images of femurs, a bone structure better than those of G1 and G2 can be seen, though there were still bubble-like structures present between the trabecular bones. The trabecular bones occupied about 62.4% of surface area. This surface area is increased by about 11.8% compared to G1; however, without statistically significant difference. The average thickness of the trabecular bones were 126 µm which is about 1.2 times of that of G1 with statistical significance. Thus, the test composition for G4 (OVX+MIA+H) exhibits ameliorative or therapeutic efficacy on osteoporosis.

For G5 (OVX+MIA+X), from the microscopic images of femurs, a bone structure better than those of G1 and G2 can be seen, though there were still bubble-like structures present between the trabecular bones. However, the number of the bubble-like structures is less than G1. The trabecular bones occupied about 60.3% of surface area. This surface area is increased by about 9.6% compared to G1; however, without statistically significant difference. The average thickness of the trabecular bones were 126 µm which is about 1.2 times of that of G1 with statistical significance. Thus, the test composition for G5 (OVX+MIA+X) exhibits ameliorative or therapeutic efficacy on osteoporosis.

### 4.4 Conclusion

The experiments in which the data of the disease model control group G1 have significant difference compare to those of the normal control group G0 were chosen for a comprehensive analysis. The results are shown in Table 7 below.

**Table 7: Results of a Comprehensive Analysis of Experiments**

| | **Group** | **1** | **2** | **3** | **4** | **5** |
|---|---|---|---|---|---|---|
| | **Test Composition** | **Disease Control** | **L** | **M** | **H** | **X** |
| ***Arthritis Model*** | | | | | | |
| Hind limb supporting force (2 items) | | - | **1^{a}** | **2** | **2** | **2** |
| Histopathology analysis (5 items) | | - | **0** | **0** | **3** | **4** |
| **Therapeutic Score^{b}** | | - | **1** | **2** | **5** | **6** |
| ***Osteoporosis Model*** | | | | | | |
| Micro-computed tomography image analysis (6 items) | | - | **0** | **0** | **0** | **6** |
| Histopathology analysis (2 items) | | - | **0** | **1** | **1** | **1** |
| **Therapeutic Score** | | - | **0** | **1** | **1** | **7** |
| **Therapeutic Index^{c}** | | - | **1/15** | **3/15** | **6/15** | **13/15** |

| | | | | | | |
|---|---|---|---|---|---|---|
| ^{a}: The number of analytical items that shows statistical significant therapeutic or ameliorative effect compared to G1. One significant change scores 1 point. ^{b} :In the disease model, the total number of analytical items that shows statistical significant therapeutic or ameliorative effect in the tested groups. ^{c} :Therapeutic index = the sum of the therapeutic scores of all disease model/the sum of the analytical items of the disease models. | | | | | | |

In the experimental conditions, the test compositions were orally administered for 28 days. The results show that Composition X has efficacies in ameliorating arthritis pain, repairing the tissue damage caused by arthritis, increasing bone mass density, and increasing the thickness and number of trabecular bones. Composition H has efficacies in ameliorating arthritis pain, repairing the tissue damage caused by arthritis, and increasing the thickness of trabecular bones. Composition M has efficacies in ameliorating arthritis pain and increasing the thickness of trabecular bones. Composition L has efficacy in slightly ameliorating arthritis pain. The therapeutic efficacies are in the following order: Composition X > Composition H > Composition M > Composition L.

### Example 5: Evaluations of the Anti-Oxidative Efficacies of the DNA Extracts

### 5.1 Evaluation for efficacies in clearing NO free radicals

Standard samples for NO free radicals were prepared by adding 8 µl NaNO₂ (10 mM) to 492 µl H₂O to form a 160 µM NaNO₂ solution. 250 µL of the 160 µM NaNO₂ solution was added into an eppendorf containing 250 µL H₂O to reach a concentration of 80 µM and serially diluted into 40 µM, 20 µM, 10 µM, and 5 µM and using pure water as 0 µM for measuring a standard curve of NO concentrations.

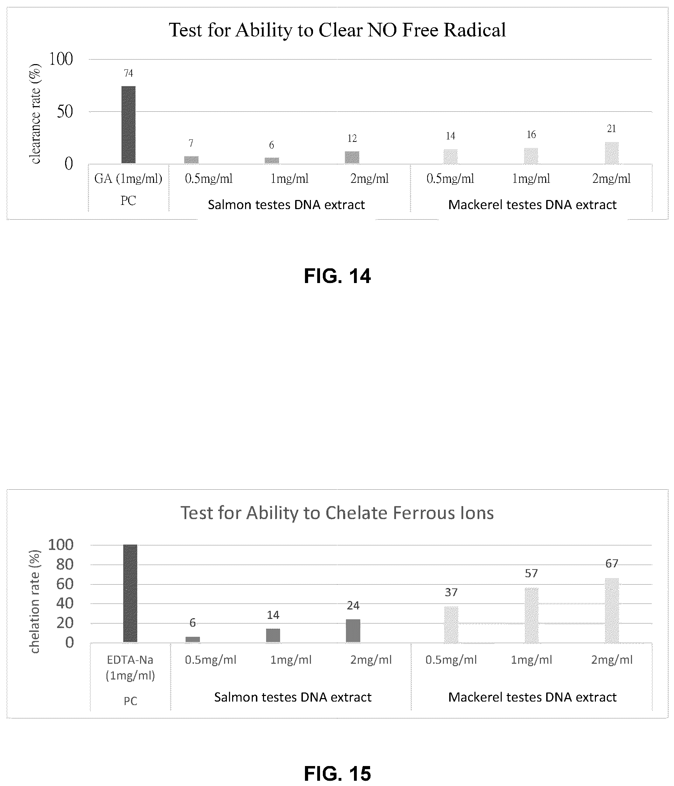

For test sample analysis, each 50 µL of 10 mM sodium nitroprusside (SNP) or H₂O (blank) was added with 50 µL 1 mg/ mL sample or Gallic acid (GA) (positive control) and pre-incubated for 25-60 min under room temperature. After the pre-incubation, 50 µL 2% sulfanilamide (SA) and 50 µL 0.2% N-(1-Napthyl) enthylenediamine dihydrochloride (NED) were added to the test samples and incubated for 10 min without light under room temperature. The samples were detected under OD 540 nm. The results are shown in Figure 14, which shows that DNA extracts from salmon or mackerel testes exhibit NO scavenging efficacies, wherein mackerel DNA extracts demonstrated superior efficacies.

### 5.2 Evaluation for efficacies in chelating ferrous ions

90 µL of the DNA extract sample or positive control (1 mg/ mL EDTA-2Na ) was treated with 20 µL 0.3 mM FeCl₂•4H₂O and then added with 40 µL 0.75 mM ferrozine or H₂O (blank) followed by incubation for 10 min under room temperature. The samples were detected under OD 562 nm. The results are shown in Figure 15, which shows that DNA extracts from salmon or mackerel testes exhibit ferrous ions chelating efficacies, wherein mackerel DNA extracts demonstrated superior efficacies.

### Example 6: Evaluations of the Anti-depressant Efficacies of the DNA Extracts

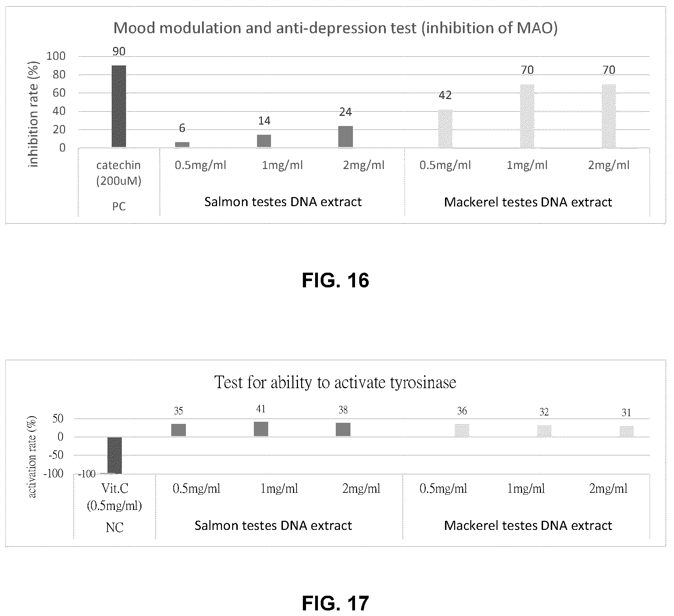

The DNA extracts were evaluated for efficacies in inhibiting monoamine oxidase (MAO), an enzyme known to be associated with depression. 50 µL sample or positive control (200 µM (+)-Catechin ) was treated with 50 µL 0.1 mg/ mL MAO-A and then added with 100 µL 500 mM Kynuramine to form a reaction mixture. The mixture was incubated for 40 min without light under 37°C. After incubation, the mixture was added with 10 µL Amplex Red (0.5 mM) and then 20 µL horseradish peroxidase (HRP) (1 mg/mL) or H₂O (blank). The mixture was then further incubated for 5 min without light under room temperature. 0.3 mL H₂O was then added before detection under OD 571 nm. The results are shown in Figure 16, which shows that DNA extracts from salmon or mackerel testes exhibit MAO inhibitory efficacies, wherein mackerel DNA extracts demonstrated superior efficacies.

### Example 7: Evaluations of the Black Hair Promoting Efficacies of the DNA Extracts

Tyrosinase is a key enzyme known to be involved in the formation of melanin, an important pigment for black hair formation. The efficacies of the DNA extracts in increasing tyrosinase activities were evaluated. 450 µL 0.1M pH 6.8 phosphate buffer and 500 µL 0.03% tyrosin were added to 450 µL sample or positive control (Ascorbic acid (Vit.C), 500 ug/ml) and pre-incubated for 10 min under 37°C. The reaction solutions were then added with 50 µL 350 unit/mL tyrosinase or H₂O (blank) and further incubated for 25 min under 25°C. The samples were detected under OD 475 nm. The results are shown in Figure 17, which shows that DNA extracts from salmon or mackerel testes exhibit tyrosinase activating efficacies.

## Claims

1. DNA extract for use in treating or ameliorating osteoarthritis or osteoporosis in a subject in need thereof, wherein the DNA extract is obtained from fish testes and has DNA base pairs between 10 and 2000 bp.

2. The DNA extract for use of claim 1, wherein the DNA extract is administered orally.

3. The DNA extract for use of claim 1, wherein administration of the DNA extract reduces osteoarthritis pain.

4. The DNA extract for use of claim 3, wherein the DNA extract reduces one or more of inflammation, pannus formation, cartilage damage, and bone resorption.

5. The DNA extract for use of claim 1, wherein administration of the DNA extract increases limb supporting force.

6. The DNA extract for use of claim 1, wherein administration of the DNA extract increases trabecular bone formation, surface area, and/or thickness.

7. DNA extract for use in removing free radicals in a subject in need thereof, wherein the DNA extract is obtained from fish testes and has DNA base pairs between 10 and 2000 bp.

8. The DNA extract for use of claim 7, wherein the DNA extract is administered orally.

9. The DNA extract for use of claim 7, wherein the free radical is NO free radical.

10. DNA extract for use in treating or ameliorating depression in a subject in need thereof, comprising administering to the subject a therapeutically effective amount of DNA extract obtained from fish testes and having DNA base pairs between 10 and 2000 bp.

11. The DNA extract for use of claim 10, wherein the DNA extract is administered orally.

12. The DNA extract for use of claim 10, wherein administration of the DNA extract inhibits monoamine oxidase (MAO).

13. DNA extract for use in promoting black hair formation in a subject in need thereof, wherein the DNA extract is obtained from fish testes and has DNA base pairs between 10 and 2000 bp.

14. The DNA extract for use of claim 13, wherein the DNA extract is administered orally.

15. The DNA extract for use of claim 13, wherein administration of the DNA extract increases tyrosinase activities.
